(19) Europäisches Patentamt / European Patent Office / Office européen des brevets

(11) EP 3 029 078 B1

(12) **EUROPEAN PATENT SPECIFICATION**

(45) Date of publication and mention of the grant of the patent:
**31.01.2018 Bulletin 2018/05**

(51) Int Cl.:
***C08F 220/00*** (2006.01) ***C09J 133/00*** (2006.01)
***C07D 207/273*** (2006.01) ***C07D 209/50*** (2006.01)
***C09D 125/00*** (2006.01)

(21) Application number: **14004129.4**

(22) Date of filing: **05.12.2014**

(54) **Cyclic N,O acetal compounds and polymers derived therefrom**

Cyclische N, O-Acetal-Verbindungen und davon abgeleitete Polymere

Composés N cyclique, O acétal et dérivés polymères de ces derniers

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**08.06.2016 Bulletin 2016/23**

(73) Proprietor: **Organik Kimya Sanayi Ve Tic. A.S.**
**ISTANBUL (TR)**

(72) Inventors:
- **Acar, Ali Ersin**
  **34342 Bebek Istanbul (TR)**
- **Bayrak, Sezgin**
  **34342 Bebek Istanbul (TR)**
- **Lorenzo, Guillermo Perez**
  **34075 Kemerburgaz/Eyup, Istanbul (TR)**
- **Gökben, Banu**
  **34075 Kemerburgaz/Eyup, Istanbul (TR)**

(74) Representative: **Müller-Boré & Partner Patentanwälte PartG mbB**
**Friedenheimer Brücke 21**
**80639 München (DE)**

(56) References cited:
**EP-A1- 0 215 245 US-A- 4 788 288**

- **RAJANI BHAT ET AL: "Tunable Thermoresponsive Pyrrolidone-Based Polymers from Pyroglutamic Acid, a Bio-Derived Resource", MACROMOLECULAR RAPID COMMUNICATIONS, vol. 34, no. 5, 12 March 2013 (2013-03-12) , pages 447-451, XP055170136, ISSN: 1022-1336, DOI: 10.1002/marc.201200735**
- **X.-L. SUN ET AL: "Thermoresponsive block copolymer micelles with tunable pyrrolidone-based polymer cores: structure/property correlations and application as drug carriers", JOURNAL OF MATERIALS CHEMISTRY B, vol. 3, no. 5, 2 December 2014 (2014-12-02), pages 814-823, XP055170166, ISSN: 2050-750X, DOI: 10.1039/C4TB01494D**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 029 078 B1

## Description

The present invention relates to olefinically unsaturated monomers which can crosslink and which can be homo- and copolymerized with other ethylenic monomers to form crosslinkable polymers useful in coatings, adhesives and textile binders. The crosslinking reaction is promoted by heat and the resulting crosslinked polymers are solvent resistant, showing no formaldehyde release while curing.

Polymers obtained by the polymerization of ethylenic monomers that contain thermally curable functional groups are useful in many areas of textile, coating and adhesive businesses. Such polymers can be in the form of solid, aqueous dispersion, emulsion or solution. Polymers containing epoxy, carboxylic acid, blocked isocyanate, or N-methylol groups, are among the most widely used polymers obtained from ethylenic monomers. These groups are incorporated into the ethylenic backbone as pendant groups which in principle do not interfere with the polymerization process. In the textile industry, binders containing N-methylol acrylamide in their backbone are the most commonly used binders. However, N-methylol containing binders such as N-methylol acrylamide emit formaldehyde during the curing process.

US 3,311,642 discloses an example (Example 8) where N-(2-tetrahydropyranyl)-acrylamide (THPA) is obtained by the reaction of DHP and acrylamide. This patent relates to the use of such compounds as antifungal agents and therefore the crosslinking ability of such compound is not discussed or investigated. Other than the cyclic acetal, linear dialkyl substituted acrylamides have been disclosed in US 4,959,489. There are also few examples of cyclic acetals bound to a nitrogen such as, in US 5,672,703, US 5,783,642 and US 5,777,040 cyclic and non-cyclic acetals. In US 2011/0207052 sulfonamides bound to tetrahydro-pyranyl and furanyl are disclosed as photoresist materials.

In the following Article with the title "Tunable Thermoresponsive Pyrrolidone-Based Polymers from Pyroglutamic Acid,a Bio-Derived Resource" by Rajani Bhat and Agostino Pietrangelo, in Macromol. Rapid Commun., 2013, 34, 447, pyrolidone based polymers with changing alkoxy substituents are disclosed.

X.-L Sun et al, in "Thermoresponsive block copolymer micelles with tunable pyrrolidone-based polymer cores: structure/property correlations and application as drug carriers" in Journal of Material Chemistry B, 2015, 3, 814-815, discuss the potential of A-B type of block co-polymers, which form micellar structures that can potentially be used as drug carrier, where one of the block is made of pyrolidone substituted ethylenic repeat units.

Accordingly, the object underlying the present invention is to provide novel types of polymeric binder which upon curing do not release any formaldehyde which can therefore be considered as formaldehyde free binders.

This object and others which will become apparent from the following disclosure, are achieved by the present invention which relates to cyclic N,O acetal compounds having the general structural formula (I)

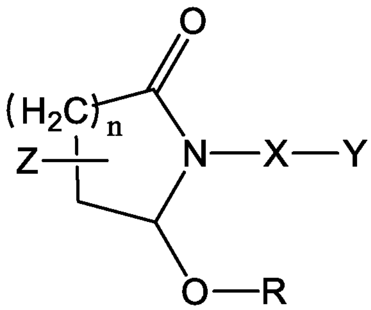

(I)

wherein

n is 1 or 2,
Y is selected from (meth)acryloyloxy and X is
an ethylene ($-CH_2-CH_2-$), propylene ($-CH_2-CH_2-CH_2-$), or butylene ($-CH_2-CH_2-CH_2-CH_2-$) moiety
R is selected from the group consisting of hydrogen, methyl, ethyl, propyl and isopropyl, and
Z, if present, is selected from an unsubstituted or substituted, annellated benzene ring, with the proviso that, if Z is present, n is 1.

Accordingly, the present invention includes new ethylenic monomers and polymers resulting therefrom which contain amido N,O acetals and hemi acetals as crosslinkable functional groups.

The cyclic N,O acetal monomers show especially rapid reactivity and efficient crosslinking. The resulting crosslinked products show excellent solvent and water resistance, low energy cure and good adhesion for application as coatings, binders or adhesives. In particular, they contain no formaldehyde.

The cyclic N,O acetal monomers of the present invention are free radical polymerizable. Accordingly, another aspect of the invention comprises polymers incorporating such compounds as polymerized monomers. Because of the

incorporated monomers, the polymers can self-crosslink under acid catalysis and/or thermosetting conditions. Moreover, they react with active hydrogen compounds such as alcohols and diols.

In another preferred embodiment of the present invention, in the above structural formula (I), n=1 and Z is absent.

In a further preferred embodiment of the present invention, in the above structural formula (I), n=1 and Z is present.

In an even further preferred embodiment of the present invention, in the above structural formula (I), n=2.

Preferably, the residue R in the above structural formula (I) is methyl, ethyl or isopropyl, more preferably methyl or ethyl.

The cyclic N,O acetal monomers of the present invention being olefinically unsaturated can be homopolymerized, or polymerized in any amount, for example, ranging from greater than 0 to over 99 wt %, with each other and/or other copolymerizable monomers. It is preferred that the copolymers contain about 0.5 to 10 wt % of the cyclic N,O acetal monomers of the present invention, especially about 1 to 3 wt % in nonwoven binder polymers.

Suitable copolymerizable monomers include monoolefinically and polyolefinically unsaturated monomers including C3 -C10 alkenoic acids, such as acrylic, methacrylic, crotonic and isocrotonic acids and their esters with C1 -C18 alkanols, such as methanol, ethanol, propanol, butanol and 2-ethylhexyl alcohol (C4 -C28 alkyl acrylates); alpha,beta-unsaturated C4 -C10 alkenedioic acids such as maleic acid, fumaric acid and itaconic acid and their monoesters and diesters with the same C1 - C18 alkanols; vinyl halides such as vinyl chloride and vinyl fluoride; vinylidene halides such as vinylidene chloride; alkenes, such as ethylene, propylene and butadiene; styrene, vinyltoluene and other substituted styrenes; and nitrogen containing monoolefinically unsaturated monomers, particularly nitriles, amides, N-methylol amides, lower alkanoic acid esters of N-methylol amides, lower alkyl ethers of N-methylol amides and allyl carbamates, such as acrylonitrile, acrylamide, methacrylamide, N-methylolacrylamide, N-methylol methacrylamide, N-methylol allyl carbamate and N-methylol lower alkyl ethers and N-methylol lower alkanoic acid esters of N-methylolacrylamide, N-methylol methacrylamide and N-methylol allyl carbamate; vinyl esters of C1 -C18 alkanoic acids, such as vinyl formate, vinyl propionate, vinyl laurate and especially vinyl acetate; vinyl ethers, such as methyl vinyl ether and isobutyl vinyl ether; and vinylamides such as N-vinyl pyrrolidone, N-vinylacetamide and N-vinylformamide. In particular embodiments, the polymer latices can be composed of n-butyl acrylate-methyl methacrylate, ethyl acrylate, 2-ethylhexyl acrylate or any other polymerizable monomer.

Vinyl acetate-butyl acrylate and butyl acrylate-methyl methacrylate terpolymers of the cyclic N,O acetal monomers of the present invention are particularly preferred.

The preferred polymers incorporating the cyclic N,O acetal monomers of the present invention are prepared with 90 to 99.5 wt % of the following comonomer systems: vinyl acetate; vinyl acetate-ethylene; vinyl acetate-butyl acrylate; vinyl chloride-ethylene; C4 to C28 alkyl acrylates such as butyl acrylate, methyl methacrylate and the like; vinyl acetate-acrylate; and styrene. In the vinyl acetate-ethylene and vinyl chloride-ethylene terpolymers, ethylene may compose 1 to 80 wt % of the polymer, preferably 5 to 40 wt %. In the vinyl acetate-acrylate terpolymers the acrylate may compose 0.5 to 99 wt % of the polymer, preferably 15 to 50 wt %. However, any other polymerizable monomer can be used.

The cyclic N,O acetal monomers of the present invention can be homopolymerized, copolymerized with each other or copolymerized with at least one of the above copolymerizable monomers by solution or aqueous emulsion polymerization techniques well known in the art. Such polymerization techniques are described in such chemistry texts as Polymer Synthesis, Vol. I and II, by S. R. Sandler and W. Karo, Academic Press, New York and London (1974), and Preparative Methods of Polymer Chemistry, Second Edition, by W. R. Sorenson and T. W. Campbell, Interscience Publishers (John Wiley and Sons), New York (1968). Solvents which are suitable for solution polymerization include toluene, isopropanol, ethanol, methanol, benzene, acetone, ethyl acetate, acetonitrile, dimethylformamide, methyl ethyl ketone and water.

The monomers in the polymerization formulation can be added all at once or metered into the polymerization reaction medium incrementally in an intermittent or continuous, preferably uniform, addition rate or any combination thereof in order to take advantage of the various polymerization reactivities of the various monomers.

Catalytically effective amounts of various free-radical forming materials can be used in carrying out the polymerization of the monomers, such as peroxide compounds like peracetic acid, benzoyl peroxide, and persulfate salt and azo compounds. Combination-type systems employing both reducing agents and oxidizing agents can also be used, i.e. a redox system. Suitable reducing agents, or activators include bisulfites, sulfoxylates, or other compounds having reducing properties such as ascorbic acid, erythorbic acid and other reducing sugars. The oxidizing agents include hydrogen peroxide, organic peroxides such as t-butyl hydroperoxide and the like, persulfates, such as ammonium or potassium persulfate, and the like. Specific redox systems which can be used include hydrogen peroxide and zinc formaldehyde sulfoxylate; t-butylhydroperoxide and erythorbic acid; hydrogen peroxide, ammonium persulfate, potassium persulfate or t-butyl hydroperoxide with sodium metabisulfite, sodium bisulfite, ferrous sulfate, zinc formaldehyde sulfoxylate, sodium formaldehyde sulfoxylate or sodium acetone bisulfite. Other free radical forming systems that are well known in the art can also be used to polymerize the monomers.

The oxidizing agent is generally employed in an amount of 0.01 to 1 %, preferably 0.05 to 0.5% based on the

weight of the monomers introduced into the polymerization system. The reducing agent is ordinarily added dissolved in an appropriate solvent in the necessary equivalent amount.

With regard to aqueous emulsion polymerization techniques, again, any of the well known emulsifying agents can be used, such emulsifying agents include ionic and nonionic surfactants such as sodium lauryl sulfate, sodium sulfosuccinate esters and amides, sulfonated alkyl benzenes, alkylphenoxy polyethoxy ethanols and other polyoxyethylene condensates.

The concentration range of the total amount of emulsifying agents useful is from less than 0.5 to 5% based on the aqueous phase of the emulsion regardless of a solids content.

Where necessary to maintain the pH of the aqueous emulsion reaction medium, typical buffering systems can be employed.

In addition to or in place of the surfactants, protective colloids such as polyvinyl alcohol and celluloses like hydroxyethyl cellulose, methyl cellulose, hydroxypropyl methyl cellulose and the like can be used as the emulsifying or stabilizing agent. When polyvinyl alcohol is added up-front or during the polymerization reaction as the stabilizing agent, the monomers typically graft onto the polyvinyl alcohol backbone.

The cyclic N,O acetal monomers of the present invention and their derived polymers are suitable for use as crosslinkers and adhesion promoting agents in paints and other coatings, adhesives for glass, wood, paper, metal, ceramics and other substrates; formaldehyde free binders for nonwoven products, medical/surgical applications, diaper cover stock, wipes, towels, apparel, carpeting, fabrics, filtration products and home furnishings. They may also be useful as co-reagents to reduce formaldehyde and/or improve adhesion and other performance factors when used with standard aminoplasts and phenoplasts.

The polymers derived from the cyclic N,O acetal monomers of the present invention are useful as binder compositions in the preparation of nonwoven products, or fabrics, by a variety of methods known to the art which, in general, involve the impregnation of a loosely assembled mass of fibers with the copolymer binder emulsion, followed by moderate heating to dry the mass. This moderate heating also usually serves to cure the binder by forming a crosslinked polymer. Before the binder is applied it can be mixed with a suitable catalyst for the crosslinking monomer. For example, an acid catalyst such as mineral acids, e.g. hydrogen chloride, or organic acids, e.g. p-toluenesulfonic acid, or acid salts such as ammonium chloride, are suitably used as is known in the art. The amount of catalyst is generally about 0.5 to 2% of the total binder resin.

The starting fiber layer or mass can be formed by any one of the conventional techniques for depositing or arranging fibers in a web or layer. These techniques include carding, garnetting, air-laying and the like. Individual webs or thin layers formed by one or more of these techniques can also be laminated to provide a thicker layer for conversion into a fabric. Typically, the fibers extend in a plurality of diverse directions in general alignment with the major plane of the fabric, overlapping, intersecting and supporting one another to form an open, porous structure.

When reference is made to "cellulose" fibers, those fibers containing predominantly $C_6H_{10}O_5$ groupings are meant. Thus, examples of the fibers to be used in the starting layer are the natural cellulose fibers such as wood pulp, cotton and hemp and the synthetic cellulose fibers such as rayon, and regenerated cellulose. Often the fibers in the starting layer may comprise the natural fibers such as wool, jute; artificial fibers such as cellulose acetate; synthetic fibers such as cellulose acetate, polyvinyl alcohol, polyamides, nylon, polyester, acrylics, polyolefins, i.e. polyethylene, polyvinyl chloride, polyurethane, and the like, alone or in combination with one another.

The fiber starting layer is subjected to at least one of the several types of bonding operations to anchor the individual fibers together to form a self-sustaining web. Some of the better known methods of bonding are overall impregnation, or printing the web with intermittent or continuous straight or wavy lines or areas of binder extending generally transversely or diagonally across the web and additionally, if desired, along the web.

The amount of binder, calculated on a dry basis, applied to the fibrous starting web is that amount which is at least sufficient to bind the fibers together to form a self-sustaining web and suitably ranges from about 3 to about 100% or more by weight of the starting web, preferably from about 10 to about 50 wt % of the starting web. The impregnated web is then dried and cured. Thus the fabric is suitably dried by passing it through an air oven or the like and then through a curing oven.

The monomers of the invention may also be useful as reactive diluents in coating compositions. In addition, the compounds may be useful for modifying or functionalizing polyvinyl alcohol, cellulosics (wood, paper, rayon, cotton), starch or sugars through formation of cyclic acetals with 1,2- or 1,3-diols.

The present invention will be further illustrated in the following examples without any limitation thereto.

## EXAMPLES

### EXAMPLE 1-2

### Monomer Synthesis

The following examples are intended to illustrate the present invention without limiting the scope thereof.

### EXAMPLE 1

### Synthesis of 2-(2-hydroxy-5-oxopyrrolidin-1-yl)ethyl acrylate (HoPEA)

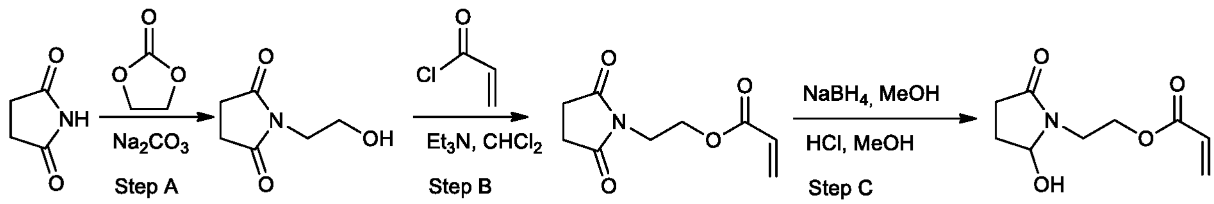


**Step A-Ethoxylation:** To an equimolar mixture of succinimide (0.5 mol) and ethylene carbonate (0.5 mol), 0.5 mol % $Na_2CO_3$ was added and the mixture was agitated at 190 °C for 1 hour. Then the reaction product was distilled under reduced pressure to obtain N-(2-hydroxyethyl)succinimide (HESI) (94%) as a white solid. $^1$H-NMR ($CDCl_3$) [ppm] : 3.68 (2H, m, -C$H_2$-O-), 3.62 (2H,m, N-C$H_2$), 3,00 (1H, br, -O*H*), 2,68 (4H, O=C-C$H_2$C$H_2$-C=O).

**Step B-Acrylation:** 27.22 g (300 mmol) acryloyl chloride was added dropwise (for 30 min) to the precooled mixture of 42.95 g (300 mmol) HESI and 33.6 g (360 mmol) triethylamine in 450 mL $CH_2Cl_2$ at 0 °C. After addition completed, reaction mixture was stirred for 3 h at room temperature. Then crude mixture was partially evaporated and the solid particles was removed by filtration. Organic mixture was first washed with 100 mL of 10 % aqueous HCl, then with 100 mL brine. Organic mixture was dried over $Na_2SO_4$ and filtered and then evaporated to dryness. Crude product was recrystallized from diethylether to obtain 2-(2,5-dioxopyrrolidin-1-yl)ethyl acrylate (DoPEA) (82 %) as a white solid. $^1$H-NMR ($CDCl_3$) [ppm]: 6.21 (1H, d, -C$H_2$=CH), 5.92 (1H, m, -$CH_2$=C*H*), 5.68(1H, d, -C$H_2$=CH), 4.17 (2H, m, -C$H_2$-O-), 3.68 (2H,m, N-C$H_2$-), 2,58 (4H, O=C-C$H_2$C$H_2$-C=O).

**Step C-Reduction:** 8.51 g (225 mmol) $NaBH_4$ was added portionwise to the precooled solution of 29.57 g (150 mmol) DoPEA in 300 mL methanol at -10 °C. At regular intervals (10-15 min) 2 mL of 1 M HCl in methanol was added into reaction mixture. After 1.5 h of agitation, the excess hydride was destroyed and reaction mixture was neutralized by addition of 1 M HCl in methanol After neutralization the crude mixture was evaporated till dryness and extracted with 200 mL $CH_2Cl_2$. The organic mixture was washed with water and dried over $Na_2SO_4$. Organic mixture was filtered through celite and then evaporated to dryness and purified by flash column chromatography using ethylacetate/$CHCl_2$ as the eluent to obtain 2-(2-hydroxy-5-oxopyrrolidin-1-yl)ethyl acrylate (HoPEA) (75 %) as a clear oil. $^1$H-NMR ($CDCl_3$) [ppm] : 6.36 (1H, dd, -C$H_2$=CH), 6.05 (1H, m, -$CH_2$=C*H*), 5.81 (1H, d, -C$H_2$=CH), 5.21 (1H, s, *C*H*), 5.02 (1H, dd, -OH), 4.24 (2H, m, -C$H_2$-O-), 3.67 (2H, m, N-C$H_2$-), 2.48-2.27 (4H,m, O=C-C$H_2$C$H_2$-C=O).

### EXAMPLE 2

### Synthesis of 2-(1-hydroxy-3-oxoisoindolin-2-yl)ethyl acrylate (HoIEA)

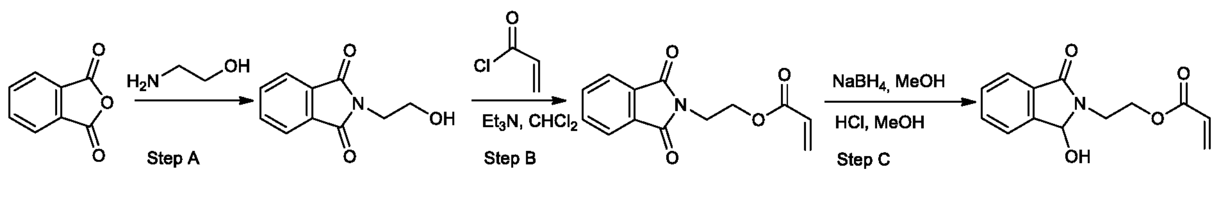


**Step A-Ethoxylation:** Equimolar mixture of phthalic anhydride (0.5 mol) and aminoethanol (0.5 mol), was

carefully heated to 80 °C for 30 min and then agitated at 200 °C for 4 hours. Then the reaction product was recrystallized from methanol to obtain N-(2-hydroxyethyl)phthalimide (HEPI) (80 %) as a white solid. $^1$H-NMR ($d_6$-DMSO) [ppm]: 7.79 (4H, m, aromatic H's), 4.82 (1H, t, O*H*), 3.60 (2H, m, -C$H_2$-O-), 3.55 (2H, t, N-C$H_2$-).

**Step B-Acrylation:** The procedure was the same as in Example 1, except that 19.1 g (100 mmol) HEPI was used in place of HESI and the crude dry product was recrystallized from acetone, methanol or purified by flash column chromatography using $CH_2Cl_2$ as the eluent to obtain 2-(1,3-dioxoisoindolin-2-yl)ethyl acrylate (DoIEA) as a white solid with 82 % yield. $^1$H-NMR ($CDCl_3$) [ppm] : 7.79 (2H, m, aromatic H's), 7.65 (2H, m, aromatic H's), 6.3 (1H, d, -C$H_2$=CH), 5.99 (1H, m, -$CH_2$=C*H*), 5.75 (1H, d, -C$H_2$=CH), 4.33 (2H, m, -C$H_2$-O-), 3.94 (2H,m, N-C$H_2$-).

**Step C-Reduction:** The procedure was the same as in Example 1, except that 36.78 g (150 mmol) DoIEA was used in place of DoPEA. the crude dry product was purified by flash column chromatography using ethylacetate/$CH_2Cl_2$ as the eluent to to yield 2-(2-hydroxy-5-oxopyrrolidin-1-yl)ethyl acrylate (HoIEA).as a clear oil with 78 % yield. $^1$H-NMR ($CDCl_3$) [ppm] : 7.49-7.57 (3H, m, aromatic H's), 7.39 (1H, m, aromatic H's), 6.28 (1H, dd, -C$H_2$=CH), 5.98 (1H, m, -$CH_2$=C*H*), 5.77 (1H, s, *C*H*), 5.75 (1H, d, -C$H_2$=CH), 4.27 (2H, m, -C$H_2$-O-), 3.63 (2H, m, N-C$H_2$-).

## EXAMPLES 3-8

### Synthesis of Latex Binders

### Apparatus

Fully automated computer controlled system equipped with 2 L jacketed glass reactor vessel, a thermostatic circulation bath, a thermocouple, three peristaltic pumps, a reflux condenser, a mechanical stirrer with stainless steel agitator were used to carry out the semi-continuous emulsion copolymerizations.

## EXAMPLE 3

### Synthesis of BA/MMA latex

Initially, reactor was charged with 175 g of DIW, 1.43 g of 28% Disponil SLS 101, 8 g of 15% Tridac Iso-40. The temperature was raised to 65 °C and mixture was agitated at 120 rpm. Monomer emulsion was prepared in 0.5 L beaker by adding 66 g of DIW, 4.29 g of Disponil SLS 101, 24 g of 15% Tridac Iso-40, 2 g of AA, 80 g of MMA and 120 g of BA under stirring. At reaction temperature 1 g of 0.4 % $FeSO_4.7H_2O$, 5 % wt of pre-emulsion, 1 g of 10 % APS and 2 g of 5 % sodium metabisulfite (MBS) were added into the reactor respectively. Upon initiation, remaining monomer emulsion was fed into reactor in 75 minutes. In parallel with monomer emulsion fed, 14 g of 5 % MBS and the mixture of 0.8 g APS and 0.7 g 25 % aqueous ammonia in 15.2 g of DIW were fed into the reactor. After the completion of feeding period, the latex was cooked for additional 15 mins and at the reaction temperature a solution of 4.2 g of 5 % *t*BHP and a solution of 9 g of 5 % MBS were added respectively to decrease the unreacted monomer content. 15 minutes after the cooking stage the system was cooled down to room temperature and the pH of the latex was adjusted to 6.5-7.0 by dropwise addition of 25 % aqueous ammonia. Solid content: 39.5 %; viscosity (Brookfield LVT 1/60): 10 cps.

## EXAMPLE 4

### Synthesis of BA/MMA/NMA (100 mmol) latex

This polymer latex is made according to the process of Example 5 with the exception that 40 g of 25 % n-methylolacrylamide is incorporated to the monomer emulsion. Solid content: 40.1 %; viscosity (Brookfield LVT 1/60): 48 cps.

## EXAMPLE 5

### Synthesis of BA/MMA/HoPEA (100 mmol) latex

This polymer latex is made according to the process of Example 5 with the exception that 19.9 g of HoPEA is incorporated to the monomer emulsion. Solid content: 41.1 %; viscosity (Brookfield LVT 1/60): 15 cps.

**EXAMPLE 6**

**Synthesis of BA/MMA/HoIEA(100 mmol) latex**

This polymer latex is made according to the process of Example 5 with the exception that 24.7 g of HoIEA is incorporated to the monomer emulsion. Solid content: 41.4 %; viscosity (Brookfield LVT 1/60): 10 cps.

**EXAMPLE 7**

**Synthesis of VAM/BA latex**

Initially, reactor was charged with 261 g of DIW 6.5 g of 31% Aerosol A 102, 8.5 g of 70% Emulsogen EPN 287 and 1 g of $Na_2CO_3$. The temperature was raised to 72 °C and mixture was agitated at 120 rpm. Monomer emulsion was prepared in 1 L beaker by adding 167 g of DIW, 3.2 g of 31% Aerosol A 102, 1.7 g of 70% Emulsogen EPN 287, 260 g of VAM and 140 g of BA under stirring. At reaction temperature 8 g of 5 % sodium metabisulfite (MBS), 1 g of 0.4 % $FeSO_4.7H_2O$, 5 % wt of pre-emulsion and 10 g of 10 % NaPS were added into the reactor respectively. Upon initiation, remaining monomer emulsion was fed into reactor in 210 minutes. After the completion of feeding period, the latex was cooked for additional 60 mins and at the reaction temperature a solution of 5 g of 7 % *t*BHP and a solution of 5 g of 7 % MBS were added respectively to decrease the unreacted monomer content. 15 minutes after the cooking stage the system was cooled down to room temperature and. Solid content: 46.3 %, pH: 4.57, viscosity (Brookfield LVT 2/60) 25 cps.

**EXAMPLE 8**

**Synthesis of VAM/BA/NMA (100 mmol) latex**

This polymer latex is made according to the process of Example 9 with the exception that 40 g of 25 % n-methylolacrylamide is incorporated to the monomer emulsion. Solid content: 47.1 %; pH: 4.62, viscosity (Brookfield LVT 2/60) 220 cps.

**EXAMPLES 9-14**

**Swell and Reticulation Index Measurements on Emulsion Polymers**

Polymer films were obtained by drying latexes at room temperature for 3 days. Triplicates from each polymer film were prepared by cutting 1 x1 cm of samples (150-200 mg) and films were cured at 160 °C for 3 and 10 min. Then, each cured film sample was placed in a jar containing 100 mL acetone. All jars were sealed and kept at room temperature for 1 day. Swollen samples were weighed and then redried at 150 °C for 20 min and reweighed for swelling and per cent soluble calculations.

$$Swell\ index :\ \frac{sample\ weight\ swollen\ in\ acetone}{original\ sample\ weight}$$

$$Solubles\ \% :\ (1-\left(\frac{weight\ of\ dry\ film\ after\ acetone\ swell}{original\ dry\ film\ weight}\right))*100$$

**Table 1. Test Results on Dry Films**

| Example | x-linker type | Latex Composition | Swelling Index (Acetone) | | % Solubles in Acetone | |
|---|---|---|---|---|---|---|
| | | | 3' | 10' | 3' | 10' |
| 9 | none | BA/MMA | ∞ | ∞ | 100 | 100 |
| 10 | NMA | | 5.9 | 5.6 | 5.0 | 4.5 |
| 11 | HoPEA | | 15.4 | 13.4 | 7.2 | 9.8 |
| 12 | HoIEA | | 6.7 | 9.5 | 4.7 | 5.9 |

(continued)

| Example | x-linker type | Latex Composition | Swelling Index (Acetone) | | % Solubles in Acetone | |
|---|---|---|---|---|---|---|
| | | | 3' | 10' | 3' | 10' |
| 13 | none | VAM/BA | ∞ | ∞ | 100 | 100 |
| 14 | NMA | | 8.7 | 6.4 | 10 | 8 |

**EXAMPLES 15-21**

**Performance Tests on Non-woven Applications**

Latices were impregnated into non-woven samples. A latex bath with 35% solid content was prepared and non-wovens were immersed into this bath (each side 20 seconds) and non-woven samples with 20 g/ $m^2$ dry pick-up were prepared. Non-woven samples were cured at 160 °C for 3 min. Triplicates of samples (each 5x15 cm) were conditioned at room environment for 24 h. Samples were immersed into water bath and kept 40 seconds (each side for 20 seconds) and then performance tests were carried out by Zwick dinamometre.

**Table 2. Zwick Performance Test Results on Non-woven Applications**

| Example | Latex Composition | Nonwoven | Wet Strength, (N) | Wet Elongation (%) |
|---|---|---|---|---|
| 15 | none | Nontreated | 11.2 | 2.6 |
| 16 | BA/MMA | Without Crosslinker | 39.3 | 5.0 |
| 17 | | NMA | 136.9 | 9.4 |
| 18 | | HoPEA | 104.4 | 11.6 |
| 19 | | HoIEA | 118.1 | 11.7 |
| 20 | VAM/BA | nontreated | 30.6 | 3.51 |
| 21 | | NMA | 101 | 4.50 |

## Claims

1. Cyclic N,O acetal compound having the general structural formula (I)

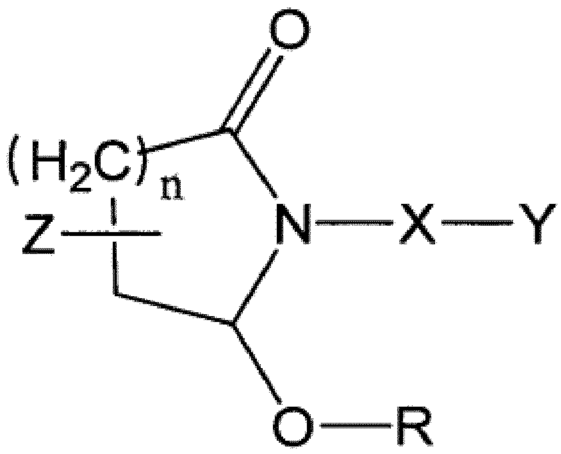

(I)

wherein

n is 1 or 2,

Y is selected from (meth)acryloyloxy and X is an ethylene (-CH2-CH2-), propylene (-CH2-CH2-CH2-), or butylene (-CH2-CH2-CH2-CH2-) moiety
R is selected from the group consisting of hydrogen, methyl, ethyl, propyl and isopropyl, and
Z, if present, is selected from an unsubstituted or substituted, annellated benzene ring, with the proviso that, if Z is present, n is 1.

**2.** Cyclic N,O acetal compound according to claim 1, wherein n=1 and Z is absent.

**3.** Cyclic N,O acetal compound according to claim 1, wherein n=1 and Z is present.

**4.** Cyclic N,O acetal compound according to claim 1, wherein n=2.

**5.** Cyclic N,O acetal compound according to any one of claims 1 to 4, wherein R is methyl or ethyl.

**6.** An emulsion polymer comprising polymeric units derived from the cyclic N,O acetal compounds according to any one of claims 1 to 5.

**7.** The emulsion polymer according to claim 6 further comprising polymeric units derived from vinyl acetate and butyl acrylate.

**8.** The emulsion polymer according to claim 6 or 7 further comprising polymeric units derived from butyl acrylate.

**9.** A cured polymer obtained by crosslinking the emulsion polymer according to any one of claims 6, 7 or 8.

**10.** Use of the emulsion polymer according to any one of claims 6, 7 or 8 or the cured polymer according to claim 9 as a coating agent, adhesive or textile binder.

**Patentansprüche**

**1.** Cyclische N, O-Acetalverbindung mit der allgemeinen Strukturformel (I)

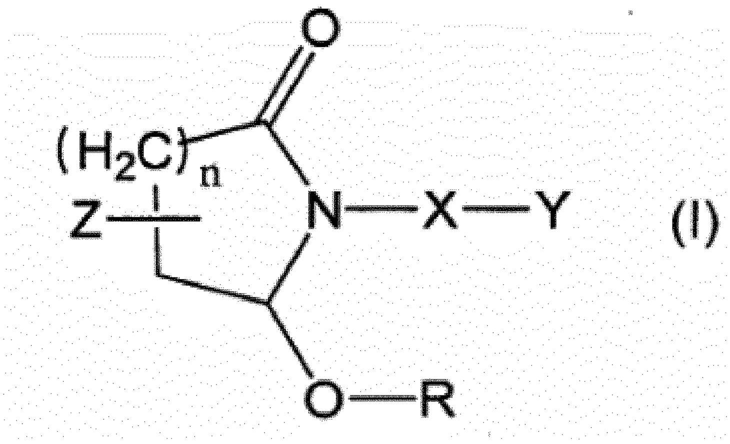

(I)

worin,

n 1 oder 2 ist,
Y ausgewählt ist aus (Meth)acryloyloxy und X ein Ethylen (-CH2-CH2-), Propylen (-CH2-CH2-CH2-) oder Butylen (-CH2- CH2-CH2-CH2-) -Gruppe
R ausgewählt ist aus der Gruppe bestehend aus Wasserstoff, Methyl, Ethyl, Propyl und Isopropyl und
Z, falls vorhanden, aus einem unsubstituierten oder substituierten, gebundenen Benzolring ausgewählt ist, mit der Maßgabe, daß, wenn Z vorhanden ist, n 1 ist.

**2.** Cyclische N, O-Acetalverbindung nach Anspruch 1, wobei n = 1 und Z nicht vorhanden sind.

3. Cyclische N, O-Acetalverbindung nach Anspruch 1, wobei n = 1 und Z vorhanden ist.

4. Cyclische N, O-Acetalverbindung nach Anspruch 1, wobei n = 2 ist.

5. Cyclische N, O-Acetalverbindung nach einem der Ansprüche 1 bis 4, worin R Methyl oder Ethyl ist.

6. Emulsionspolymer, umfassend polymere Einheiten, die sich von den cyclischen N, O-Acetalverbindungen nach einem der Ansprüche 1 bis 5 ableiten.

7. Emulsionspolymer nach Anspruch 6, ferner umfassend polymere Einheiten, die sich von Vinylacetat und Butylacrylat ableiten.

8. Emulsionspolymer nach Anspruch 6 oder 7, ferner umfassend polymere Einheiten, die sich von Butylacrylat ableiten.

9. Ein gehärtetes Polymer, erhalten durch Vernetzung des Emulsionspolymers nach einem der Ansprüche 6, 7 oder 8.

10. Verwendung des Emulsionspolymers nach einem der Ansprüche 6, 7 oder 8 oder des gehärteten Polymers gemäß Anspruch 9 als Beschichtungsmittel, Klebstoff oder Textilbindemittel.

**Revendications**

1. Composé d'acétal cyclique N, O ayant la formule générale de structure (I)

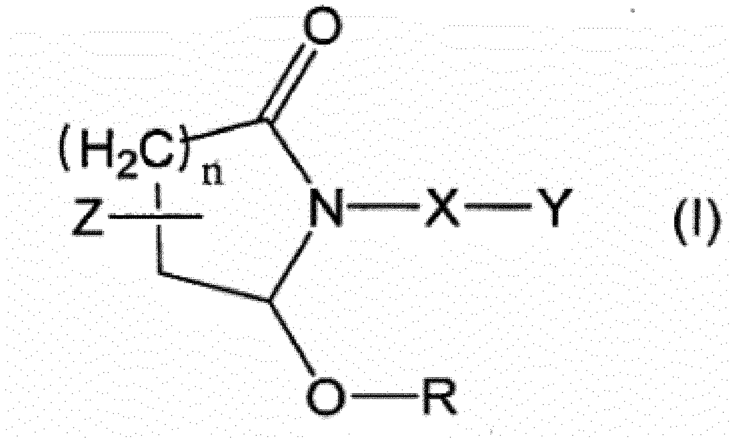

(I)

dans laquelle

n vaut 1 ou 2,
(i) Y est choisi parmi (méth)acryloyloxy et X est un éthylène (-CH2-CH2-), propylène (-CH2-CH2-CH2-) ou butylène (-CH2- CH2-CH2-CH2-),
R est choisi dans le groupe constitué par l'hydrogène, le méthyle, l'éthyle, le propyle et l'isopropyle,
et Z, le cas échéant, est choisi parmi un cycle benzène annulé non substitué ou substitué, à condition que, si Z est présent, n est égal à 1.

2. Composé cyclique d'acétal N, O selon la revendication 1, dans lequel n = 1 et Z sont absents.

3. Composé cyclique d'acétal N, O selon la revendication 1, dans lequel n = 1 et Z sont présents.

4. Composé cyclique d'acétal N, O selon la revendication 1, dans lequel n = 2.

5. Composé cyclique d'acétal N, O selon l'une quelconque des revendications 1 à 4, dans lequel R représente un groupe méthyle ou éthyle.

6. Un polymère en émulsion comprenant des motifs polymères dérivés des composés cycliques d'acétal N, O selon l'une quelconque des revendications 1 à 5.

7. Le polymère en emulsion selon la revendication 6, comprenant en outre des motifs polymères dérivés de l'acétate de vinyle et de acrylate de butyle.

8. Le polymère en emulsion selon la revendication 6 ou 7, comprenant en outre des motifs polymères dérivés de l'acrylate de butyle.

9. Un polymère durci obtenu par réticulation du polymère en émulsion selon l'une quelconque des revendications 6, 7 ou 8.

10. Utilisation du polymère en émulsion selon l'une quelconque des revendications 6, 7 ou 8 ou du polymère durci selon la revendication 9 en tant qu'agent de revêtement, adhésif ou liant textile.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3311642 A
- US 4959489 A
- US 5672703 A
- US 5783642 A
- US 5777040 A
- US 20110207052 A


### Non-patent literature cited in the description


- **RAJANI BHAT ; AGOSTINO PIETRANGELO.** Tunable Thermoresponsive Pyrrolidone-Based Polymers from Pyroglutamic Acid,a Bio-Derived Resource. *Macromol. Rapid Commun.,* 2013, vol. 34, 447
- **X.-L SUN et al.** Thermoresponsive block copolymer micelles with tunable pyrrolidone-based polymer cores: structure/property correlations and application as drug carriers. *Journal of Material Chemistry B,* 2015, vol. 3, 814-815
- **S. R. SANDLER ; W. KARO.** Polymer Synthesis. Academic Press, 1974, vol. I and II
- **W. R. SORENSON ; T. W. CAMPBELL.** Preparative Methods of Polymer Chemistry. Interscience Publishers (John Wiley and Sons), 1968